Europäisches Patentamt

(19) European Patent Office

Office européen des brevets

(11) Publication number:  **0 050 040**
**A2**

(12) **EUROPEAN PATENT APPLICATION**

(21) Application number: **81304772.7**

(22) Date of filing: **13.10.81**

(51) Int. Cl.³: **G 01 N 29/02**
**A 61 B 10/00**

(30) Priority: **14.10.80 US 196364**

(43) Date of publication of application:
**21.04.82 Bulletin 82/16**

(84) Designated Contracting States:
**AT BE CH DE FR GB IT LI LU NL SE**

(71) Applicant: **PURDUE RESEARCH FOUNDATION**
**Graduate House East**
**West Lafayette Indiana 47907(US)**

(72) Inventor: **Newhouse, Vernon Leopold**
**1800 Garden Street**
**West Lafayette Indiana 47906(US)**

(74) Representative: **Hudson, Christopher Mark**
**Erl Wood Manor**
**Windlesham Surrey GU20, 6PH(GB)**

(54) **A method and apparatus for the detection of impurities in a liquid such as blood.**

㊐ A method and apparatus are disclosed for detecting impurities in a liquid such as blood. A minimum amount of ultrasound Doppler radiation of narrow bandwidth to maximize sensitivity is directed through a transducer focused in the far field and positioned at an angle with respect to a flow of liquid, such as blood, to be monitored. The reflected radiation received at the transducer, due to backscattering agents in the liquid, is used to generate a backscatter power signal the level of which is dependent upon the scattering agents in the liquid. This signal is utilized to determined the presence of impurities in the liquid, which in the case of carbon particles in blood, provide a higher average power level than the scattering agents in pure blood.

## A METHOD AND APPARATUS FOR THE DETECTION
## OF IMPURITIES IN A LIQUID SUCH AS BLOOD

This invention relates to a method and apparatus for detecting impurities in a liquid, and more particularly, relates to detecting impurities, or contaminants, in a flow of blood.

If is often desirable, and in some cases necessary, that impurities in a liquid be quickly determined. Such is the case, for example, where impurities or contaminants are present, or introduced into, the blood of a human. Obviously, only early detection of such impurities or contaminants can lead to early commencement of corrective action.

Since blood in a human is constantly flowing, an accurate measurement of the parameters or the conditions of the blood has not been found to be an easy task. It has been found, however, that ultrasound Doppler radiation can be effectively utilized to measure blood flow as well as to detect the presence of bubbles in a flow of blood.

Continuous Doppler has been utilized before when it was not necessary to distinguish flow at one range from flow at another range. When distinguishing range is necessary, pulsed Doppler has been found to be effectively utilized since pulsed Doppler can be used without loss of sensitivity other than that expected from the smaller duty cycle of the transmitted signal used in the particular type of system.

Ultrasonic Doppler measurements depend on the return of ultrasonic power from scatterers of different types. In blood, the signals returned to the transducer have been shown to be due to the individual red blood cells, which act as scattering agents and are sufficiently numerous and acoustically different from plasma to backscatter a detectable amount of power.

The use of Doppler techniques provides a better signal-to-noise ratio over normal pulse echo techniques when utilized for particle detection, and since impurities in the blood move at approximately the same velocity as the blood cells, the echoes returned from the blood-impurity mixture can be separated from echoes due to stationary targets. This greatly increases the effective resolution of the system and makes it possible, for example, to detect and analyze blood echoes from blood flowing in vessels whose lumen is too narrow to be resolved by an ultrasound imaging system. Also, small changes in blood reflectivity can be measured by Doppler techniques, even though the power returned from blood may be 60 dB lower than simultaneous echoes returned by stationary targets.

While ultrasound Doppler has been used in the past mainly for blood velocity measurements, work has been performed using Doppler techniques to detect air bubbles where such bubbles also act as scattering when in the blood. While the detection of bubbles in the blood is necessary, it is also often necessary to detect other impurities such as charcoal particles, zeolite slurry, and/or blood clots. Such detection is important, for example, for monitoring extracorporeal blood pumps, dialysis, open heart surgery, and blood detoxification. The need for a method and apparatus

0050040

for detection of carbon particles in blood is particularly important where such detection can be used to monitor blood haemoperfusion by activated charcoal columns.

This invention provides an improved method and apparatus for detecting impurities in a liquid, and particularly for detecting impurities in blood, as well as a method and apparatus for detecting impurities such as charcoal in a flow of blood.

Specifically this invention provides a method for detecting impurities in a liquid wherein:

ultrasound Doppler radiation of narrow bandwidth is directed at flow of liquid and the radiation reflected from the scattering agents in the liquid is received;

the power levels of the received reflected radiation due to the scattering agents is found;

from the power levels, impurities in the liquid are determined;

and a device for detecting impurities in a liquid wherein the device comprises:

ultrasound Doppler means including transducer means for directing radiation of narrow bandwidth towards a flow of liquid and receiving reflected radiation therefrom, the Doppler means providing an output signal having a component indicative of impurities sensed in the flow of liquid; and

analyzing means for receiving the output signal from the Doppler means, for determining the presence of the impurities indicative of impurities included in the liquid and providing an indication of the presence of the impurities in the flow of liquid.

This invention therefore provides an improved method and apparatus for detecting impurities in a liquid such as blood.

This invention also provides an improved method and apparatus for detecting impurities such as charcoal in blood.

Additionally, this invention provides an improved method and apparatus for detecting impurities in a liquid such as blood by directing ultrasound Doppler radiation of narrow bandwidth toward a flow of blood through a transducer focused in the far field and positioned at an angle with respect to the flow of blood.

With these and other aspects in view, which will become apparent to one skilled in the art as the description proceeds, this invention resides in the novel construction, combination, method and arrangement of parts substantially as hereinafter described, and more particularly defined by the appended claims, it being understood that such changes in the precise embodiment of the herein disclosed invention are meant to be included as come within the scope of the claims.

The accompanying drawings illustrate a complete embodiment of the invention according to the best mode so far devised for the practical application of the principles thereof, and in which:

FIGURE 1 is a schematic illustrative view showing a measuring cell;

FIGURE 2 is a block diagram of apparatus for detection of impurities in blood;

FIGURE 3 is a graph showing carbon particle size characteristics;

FIGURE 4 is Doppler spectra (power v. frequency) using continuous Doppler in detecting impurities (carbon) in blood;

FIGURE 5 is Doppler spectra (power v. frequency) utilizing pulsed Doppler in detecting impurities (carbon) in blood; and

FIGURE 6 is a graph of backscattered power normalized to blood.

As brought out above, Doppler systems have previously been described for flow measurement and for observing bubbles in blood. Doppler systems are advantageous with respect to pulse echo system for this type of application.

If a pulse echo system were used, very short transmitted pulses would have to be used to image bubbles. Short transmitted and displayed pulses, however, have a large bandwidth so that the output energy of the Doppler system is spread over a large bandwidth which degrades the signal-to-noise ratio. But if a Doppler system is used for observing, the output bandwidth can be much smaller. It is not necessary to resolve the bubbles, merely detecting the presence of moving high intensity sound reflectors is sufficient. Thus, the output bandwidth is reduced with a corresponding improvement in system signal-to-noise ratio.

Another utilization of Doppler systems is that a Doppler system output corresponding to echoes from stationary targets (such as blood vessel walls, for example) occurs at zero frequency (i.e., at DC) and thus can be removed from consideration by the use of simple filters. It is therefore not necessary when using a Doppler system to transmit ultrasound bursts which are short enough to image blood without also imaging blood vessel walls.

Thus, in a Doppler system, relatively long ultrasound bursts can be transmitted and this has the advantage of reducing the peak power intensity which is required. The reason for this is that to obtain a given overall system sensitivity, it is necessary to transmit a given amount of average energy. If this energy is transmitted in relatively long bursts, the peak transmitted energy can be relatively low. However, if the bursts have to be kept short so as to image blood only without simultaneously imaging blood vessel walls, then the transmitted peak intensity must be large which is difficult to attain and may also cause tissue damage.

In an artificial portable kidney, blood is pumped past the dialysis membrane which separates it from dialysate liquid containing a slurry of fine particles of materials such as carbon and zeolite. In case of an accidental break in the dialysis membrane, slurry could enter the blood with consequent possible damage to the user of the artificial kidney. Through use of this invention, such a slurry can be monitored by an ultrasound Doppler system (which, if desired, can be simultaneously used for measuring the velocity of the blood flow) to detect such contaminants in blood.

To detect the minimal possible amount of potential slurry contamination, the output signal-to-noise in the Doppler system must be made good enough so that the variations of blood cell arrangement are larger than the variations in the Doppler system output signal due to the thermal noise of the Doppler system amplifer. In other words, the output signal-to-noise ratio must be good enough so that the blood echo variance is detectably greater than the output thermal noise.

Consider a pulsed Doppler system transmitting bursts of ultrasound of amplitude A, center frequency $f_o$, and duration $\tau$ at repetition frequency $f_r = 1/T$. The time averaged one-sided spectral density of this transmitted signal is:

$$S_t(f) a \frac{A^2 \tau^2}{2T^2} \sum_{-\infty}^{\infty} sinc^2(\tau n f_r) \; \delta\left[f - (f_o + n f_r)\right] \qquad (1)$$

Most of the energy of this spectrum is contained in $1/\tau f_r$ lines within a frequency range $2/\tau$ centered at $f_o$.

If the system is illuminating a single scatterer moving with velocity component v along the beam direction, then the echo spectrum will be identical with the of equation (1) except that a spectral line at frequency $n f_r$ will be shifted to the frequency $n f_r(1-\alpha)$, where $\alpha = 2v/c$. In typical Doppler systems, the echo spectrum will be multipled by the reference signal $cos2\pi f_o t$ and low pass filtered producing the downshifted spectrum at the output of the multiplier of form:

$$S_t(f) \alpha \frac{A^2 \tau^2}{2T^2} \sum_{-\infty}^{\infty} \text{sinc}^2(\tau n f_r) \delta(f - \{n f_r(1-\alpha) + f_o\}) \quad (2)$$

The spectrum is seen to consist of lines at the frequencies $\alpha f_o, f - \alpha f_o, f_r + \alpha f_o, 2f_r + \alpha f_o$ etc. The output of the multiplier is typically range gated which has the effect of broadening each of the spectral lines to a finite breadth, and is then passed through a sample and hold circuit which eliminates all but the lowest frequency spectral line of equation (2). The output of the sample and hold circuit is a continuous signal of center frequency $f_d = \frac{2v}{c} f_o$ and of bandwidth $B_d$. The magnitude of $B_d$ is known to be the inverse of the transit time of the scatterer through a measuring cell. The dimensions of such a measuring cell are known to be determined by the dimensions of the ultrasound beam and by the length of the transmitted and gating signals.

If the receiver thermal noise is assumed to be white, the average noise power referred to the input of the receiver is $N_o B_N$. Here $B_N$ is the input equivalent noise bandwidth and $N_o$ is the one-sided noise spectral density. Upon multiplication with reference frequency $f_o$, this noise will be transferred to the baseband frequencies such that the average output noise power equals $2N_o B_d$. Thus the effective signal-to-noise ratio enhancement of the Doppler system is:

$$SNRE = \frac{SNR_{IN}}{SNR_{OUT}} = \frac{P_{OUT}}{P_{IN}} \frac{N_{IN}}{N_{OUT}} = \left[\frac{A^2 \tau^2}{2T^2} \frac{2T}{A^2}\right]\left[\frac{N_o B_N}{2N_o B_d}\right] = \frac{1}{2} \frac{\tau}{T} \frac{B_N}{B_d} \quad (3)$$

Equation (3) shows that the SNRE of a Doppler system is equal to the bandwidth compression $B_N/B_d$, usually of the order $10^3$, multipled by the duty cycle of the transmitted signal, which is to be expected, since as $\tau/T$ goes down, the average transmitted power decreases. With a continuous Doppler, the duty cycle becomes unity. In a non-directional Doppler system as analyzed above, noise from above and below $f_o$ is brought into the output band by the multiplier. In directional Doppler systems, only noise from one side of $f_o$ is brought into the passband which doubles the SNRE. Hence for a directional continuous Doppler:

$$SNRE = B_N/B_d \qquad (4)$$

Of course, in a continuous Doppler system, it is difficult to assign a value to the input bandwidth $B_N$. However, one can establish the advantage in output signal-to-noise ratio produced by a Doppler system compared to the equivalent range resolving pulse echo system by the following argument. Assume that the pulse echo system uses pulses $\tau$ seconds long. The measuring cell of this system is $\frac{\tau c}{2}$, where c is the velocity of sound. The output equivalent noise bandwidth $B_N$ is $1/\tau$hz. For the equivalent continuous Doppler system, the transit time of particles through the measuring cell is $t_\tau = \frac{\tau c}{2v}$. The baseband bandwidth $= 1/t = \frac{2v}{\tau c}$. Thus, the signal-to-noise ratio advantage of the Doppler system over the pulse echo system of equivalent effective resolving power is:

$$SNRA = \frac{1/\tau}{2v/\tau c} = \frac{c}{2v} \qquad (5)$$

which is usually of order $10^3$.

The ratio of the power reflected by blood cells and carbon particles can be predicted from knowledge of their relative numbers and the relative numbers and the relative differential cross section ($\sigma_d$). There are two equations which have generally been used to calculate $\sigma_d$. The most accurate takes into account the viscosity of the medium. The increased precision of using this equation is not justified when compared with the experimental error of the method of this invention, so a simpler equation may be used. For a viscous particle surrounded by a frictionless environment:

$$\sigma_d = \frac{1}{9}k^4 a^6 \left[ \frac{K_e - K}{K} + \frac{3p_e - 3p}{2p_e + p} \cos \theta \right]^2 \qquad (6)$$

where

$K_e$ = adiabatic compressibility of the particle

$K$  = adiabatic compressibility of the medium

$p_e$ = density of the particle

$p$  = density of the medium

$\theta$  = scattering angle, or angle between incident beam and receiving beam (180° where the transmitting and receiving transducers are encased next to each other in the probe

$a$  = particle radium.

When the backscattered power is assumed to be equal to the sum of the powers backscattered by each particle, the power received by the transducer is:

X-5628        -11-

$$P = \frac{nVk^4a^6I_iA}{9r^2} \left[ \frac{K_e-K}{K} + \frac{3p_e-3p}{2p_e+p} \cos\theta \right]^2 \quad (7)$$

where

$n$ = number of particles per $cm^3$

$V$ = volume of fluid observed

$r$ = distance from transducer to this volume

$I_1$ = incident power

$A$ = area of receiving aperture

$k$ = $2\pi$/wavelength

It has been found that at a hematocrit of 40, the backscattered power of blood is only about one fifth the power obtained by assuming independent scatterers. A correction factor $A_d$ has therefore been introduced to account for this effect. This effect is known to occur for very heavy concentration of scatterers such as is provided by the erythrocytes in normal blood. For the much lower concentrations of carbon particles, no correction factor should be required. Thus, the corrected backscattered power may be written as $P_{erythrocytes} = P_e = A_dP$, where $A_d \approx 0.2$ for a hematocrit of 40.

From this information an equation can be derived for the ratio of power backscattered from red blood cells in a mixture of both, i.e.,

$$\frac{P_c}{P_e} = \frac{n_c}{A_c n_e} \left[ \frac{a_c}{a_e} \right]^6 \left[ \frac{\frac{K_c-K}{K} + \frac{3p_c-3p}{2p_c+p} \cos\theta}{\frac{K_e-K}{K} + \frac{3p_e-3p}{2p_e+p} \cos\theta} \right]^2 \quad (8)$$

In this equation, the subscripts c and e refer to carbon and erythrocytes, respectively. The values of the constants are shown in Table 1.

## TABLE 1

Acoustic Properties of Blood and Carbon

| | Density $(gm/cm^3)$ | Adiabatic Compressibility $(cm^2/dyne)$ | Concentration $(\#/cm^3)$ | Average Radius |
|---|---|---|---|---|
| Erythrocyte | 1.092 | $34.1 \times 10^{-12}$ | $5 \times 10^9$ (Ht = 40) | 2.75 |
| Carbon | 1.76 | $2.6 \times 10^{-12}$ | | |
| Plasma | 1.021 | $40.9 \times 10^{-12}$ | | |

In the presence of carbon particles of different radii, the term $a_c^6$ in equation (3) should be replaced by

$$\overline{a_c^6} = \Sigma a_{c_i}^6 / \Sigma\, n_{c_i} \qquad (9)$$

where $n_{c_i}$ is the concentration of carbon particles of radius $a_{c_i}$.

The Doppler system must use a minimum amount of transmitted ultrasound energy so as to minimize the risk of cell damage. An additional advantage is that energy drain is also minimized where the system is powered by a battery. It is therefore important to maximize Doppler system sensitivity.

Assume that an ultrasound Doppler flow measurement system observes uniform velocity flow. In that case, the spectrum of the Doppler system output signal will consist of a noise "floor" at all frequencies plus extra energy in a spectrum of width $B_d$ centered at the Doppler frequency $f_d$. If a sufficiently long observation time T is available, the Doppler output spectrum can be analyzed to within a resolution $\Delta f = 1/T$. If the peak spectral energy density of the Doppler portion of the spectrum is $S_p$, then

$$S_p \times B_d = E \qquad\qquad (10)$$

where E is the backscattered energy received by the Doppler system transducer from the moving targets. Changes in the scatterer density will lead to changes in the peak Doppler spectral density $S_p$ and these changes can be measured more accurately the larger the ratio $S_p/N$ where N is the spectral density of the noise floor. The sensitivity of the system for a given transmitted power can thus be increased if $S_p$ can be increased. From the equation given above this can be achieved for constant transmitted energy and therefore for constant backscattered energy E by making $B_d$ smaller. (The sensitivity would increase until $B_d$ becomes smaller than the attainable frequency resolution $\Delta f$). In summary, the sensitivity of the Doppler system is maximized if $B_d$ is minimized.

For a Doppler system observing uniform velocity flow, $B_d$ is minimized if $\Delta \phi$ can be minimized

X-5628                                    -14-

($\Delta\phi$ is the largest angle over which rays transmitted by the transducer can be backscattered to the transducer).

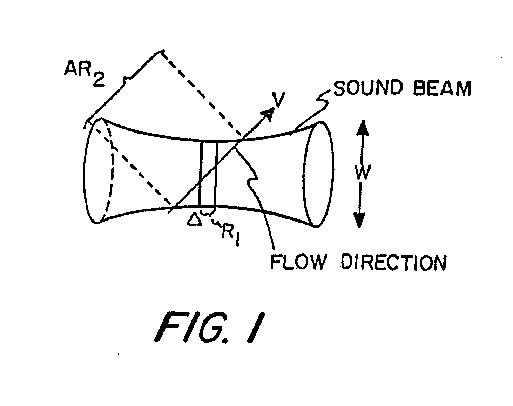

For a transmitted pulse of length $\tau$ whose echo is strobed with an infinitely narrow pulse, the length along the beam from which measurements are obtained called the measuring cell, is $\Delta R = \frac{1}{2}c\tau$ where c is the velocity of sound. If the transmitted burst length $\tau$ is smaller then the transit time of scatterers passing through the measuring cell is determined by the ends of the cell. However, if the transmitted burst length $\tau$ is large, the transit time is determined by the edges of the ultrasound beam. These two cases are shown in FIGURE 1 where $\Delta R_1$ represents the case of the small measuring cell, and $\Delta R_2$ that of the large measuring cell. The smallest $B_d$ is always obtained for case 2 where the transit time is determined by the edges of the ultrasound beam rather than by the ends of the measuring cell.

$\Delta\phi$ approaches a minimum value of order $\lambda/D$ where $\lambda$ is the ultrasound wavelength and D the transducer diameter in the very far field of unfocused transducers. However, this situation has not been considered desirable for flow measurement because of its poor spatial resolution. The geometry which minimizes both $\Delta\phi$ and therefore $B_d$ as well as providing good spatial resolution, was found to be that of a beam focused in its far field. Under those circumstances the output fractional bandwidth

$$\frac{B_d}{f_d} \doteqdot \frac{D}{F} \tan \theta \qquad (11)$$

where F is the focal length of the lens and θ the angle between the flow and the beam axis. For F in the far field of the transducer, the fractional bandwidth given in this equation approaches its minimum possible magnitude of order $\lambda/D$.

Thus, the sensitivity of Doppler system is maximized by operating the system with transducers focused in the far field which minimizes the Doppler signal output bandwidth and thus maximizes the sensitivity. When observing flow in relatively small blood vessels, the width of the focal spot in the ultrasound beam must be kept as small as possible so as to minimize output spectrum broadening due to velocity gradients. Since for very long focal lengths the focal spot diameter becomes relatively large, the minimum Doppler output spectrum in these cases is obtained by choosing a focal length which is a compromise between having small range cells and thus small velocity gradient Doppler broadening effects and having a very long focal length which would allow the Doppler spectrum bandwidth to approach its minimum value of $\lambda/D$ for uniform velocity flow. Present Doppler systems use transducers which are usually focused in the near field or use transducers with flow measured in the near field which are not focused, and which give much bigger values of $B_d$ than is ideally possible. Using the techniques described herein, these systems obtain considerably smaller values of $B_d$ and a correspondingly higher sensitivity.

To experimentally test the method and apparatus of this invention, movement of blood through a hemodialysis system was duplicated. The insonification of the blood was done while the blood was made to flow through a 3/8" ID silicone tube connecting two 500 ml. Erlenmeyer flasks at different heights. The connecting tubing was submerged in a water bath, and each flask was equipped with a magnetic stirrer to keep the erythrocytes and carbon from settling.

A Parks, Inc. 10 MHz continuous directional Doppler flowmeter was initially used for measurement. Similar results were obtained with a 2.25 MHz pulsed Doppler system.

The transducer probe of the Doppler flowmeter contained both the transmitting and receiving crystals, and this probe was placed in the water bath about 3 cm from the silicone connecting tubing and at an angle of about 45° with respect to it.

Outdated human blood, warmed to about 30°C and filtered through a 60µ filter to remove large contaminants such as blood clots, was placed into the upper of the two Erlenmeyer flasks and allowed to flow through the tubing.

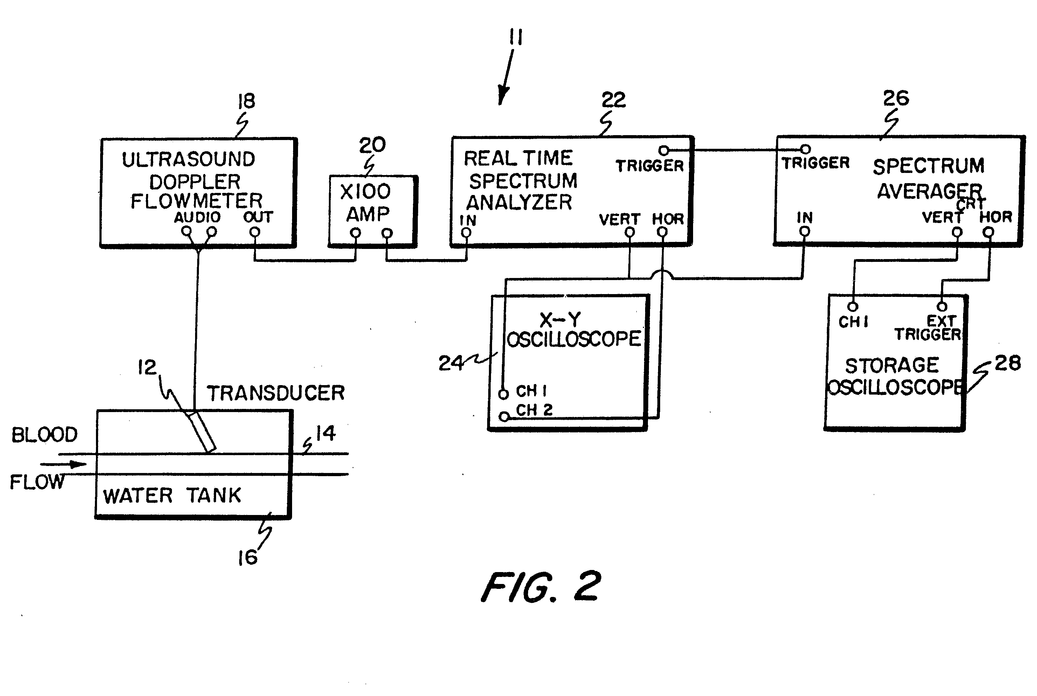

The electronic system 11 for monitoring the blood flow is shown in FIGURE 2. Ultrasonic Doppler radiation is directed from transducer 12 at an angle toward the blood flowing through tube 14 (immersed in water both 16 as shown in FIGURE 2). The reflected signal is received by transducer 12 and coupled therefrom to Doppler flowmeter 18 (either a Parks meter or pulsed Doppler system). The reflected signal is demodulated by the flowmeter and coupled to amplifier 20 where the signal indicative of reflected backscattered power is amplified and then coupled to real time spectrum analyzer 22. Analyzer 22 receives the data and converts it into a power spectrum every 50 ms.

The output of the spectrum analyzer is viewed by an oscilloscope 24 and is also applied to spectrum averager 26. Spectrum averager 26 receives a set number of spectra and then calculates their average. In this way, the ransomness of the blood echo is minimized by averaging over a certain time period. A second oscilloscope 28 records the output of spectrum averager 26.

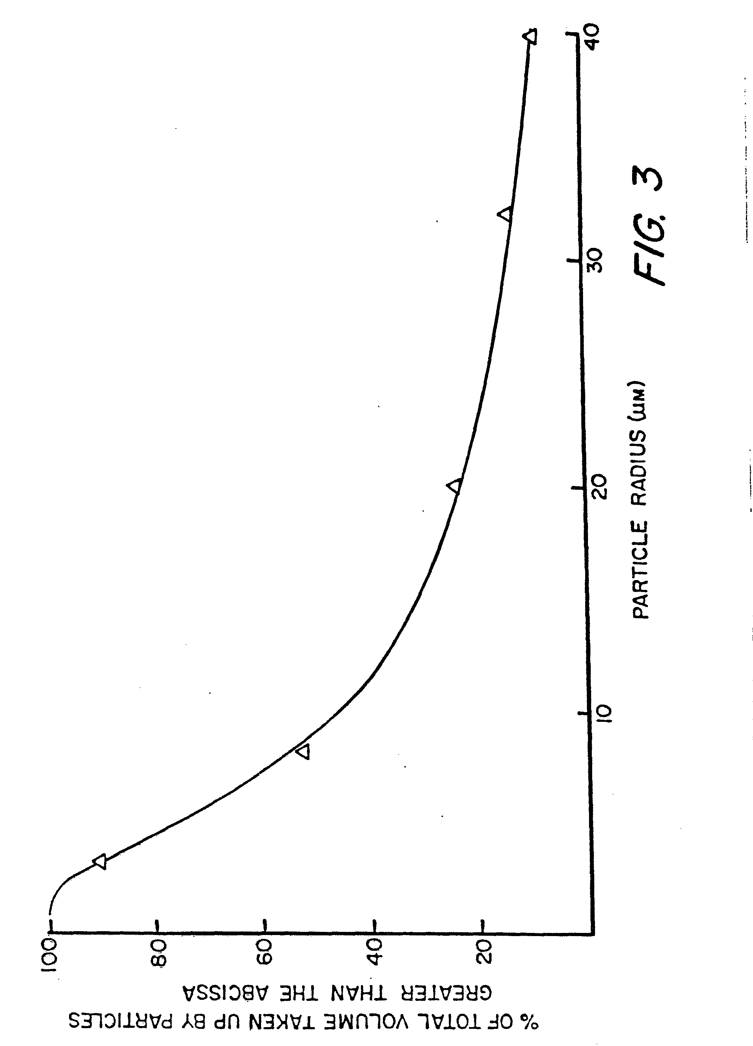

The blood caused to flow through tube 14 was first free of carbon particles and then later had different size carbon particles added thereto. The size distribution of the carbon particles used was estimated using a Coulter counter which uses screens to sift out particles of given sizes, and counts these particles. The size distribution of the carbon particles utilized as obtained with the Coulter counter is shown in FIGURE 3.

Averages of 32 spectra were obtained. First, pure blood was allowed to flow through tubing 14 while a spectrum was obtained, then (when about 300 ml of blood was left in the upper flask) a small amount of carbon suspension was added. The spectrum of the scattering from the blood contaminated with carbon was then obtained. This procedure was repeated for different concentrations of carbon. For each different carbon concentration, the entire procedure required about 2 to 3 minutes, once the pure blood started to flow. It was felt that in this short time, the effect of the blood changing its properties due to exposure and handling would be minimal.

Similar experimental work has been carried out utilizing blood from an animal (dog) being dialized, which blood was routed through tubing in a water bath past a transducer in the same manner set forth above. This blood was analyzed with an electronic system like that shown in FIGURE 2 except that a power averaging circuit was used instead of a spectrum analyzer. This circuit provided an output proportional to the average backscattered power, integrated over one second. The backscattered echo of the pure blood of the dog was recorded over many dialysis cycles to get a baseline for future comparison. Then 60 ml of blood was withdrawn from the dog, and the dog was cut off from the Dialyzer. This 60 ml of blood was allowed to go through several dialysis cycles (to make sure it was representative of pure blood), and then a known amount of carbon was added to it. This was repeated several times, with additional increments of carbon.

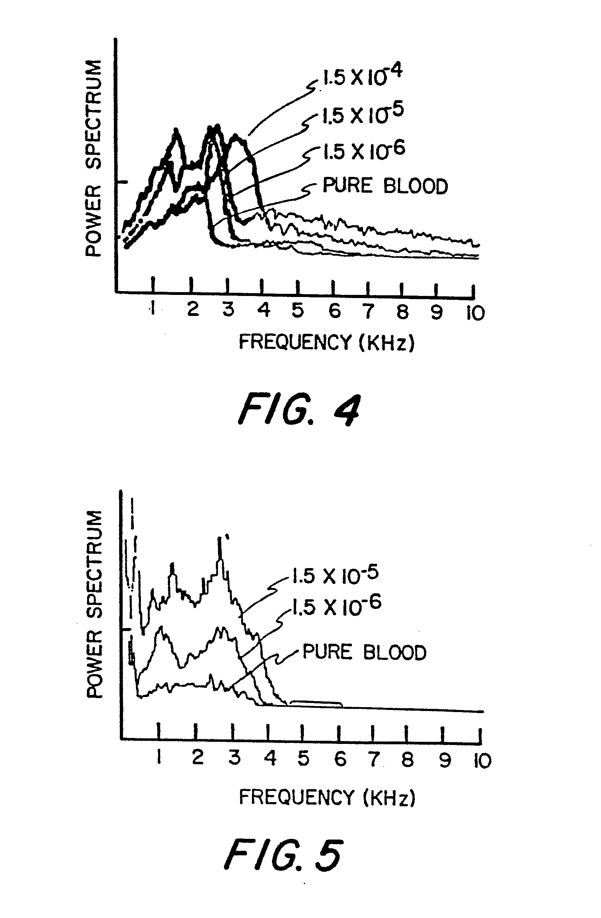

FIGURE 4 shows a Doppler spectrum (power vs. frequency) utilizing continuous Doppler for pure blood as well as a series of Doppler spectra obtained with increasing amounts of carbon ($1.5 \times 10^{-6}$ gm carbon/ml of blood; $1.5 \times 10^{-5}$ gm carbon/ml of blood; and $1.5 \times 10^{-4}$ gam carbon/ml of blood). FIGURE 5 shows similar spectra using pulsed Doppler (pure blood; $1.5 \times 10^{-6}$ gm carbon/ml of blood; and $1.5 \times 10^{-5}$ gm carbon/ml of blood).

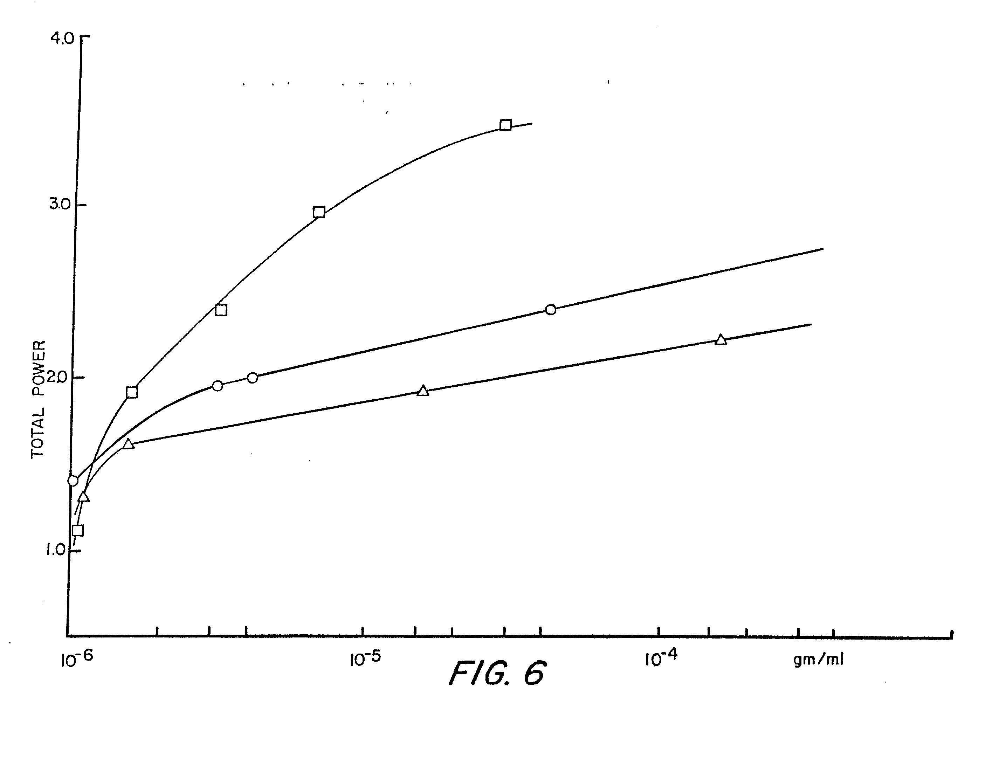

The data obtained from the Doppler spectra is shown graphed in FIGURE 6 as a function of carbon concentration. FIGURE 6 shows that the power detected by the receiving transducer increased with increasing carbon concentration, and the minimum amount of carbon detectable was found to be $1.5 \times 10^{-6}$ gm carbon per ml of blood. This was found to be true both in vivo and in vitro.

To compare this with theory, equations 8 and 9 (set forth hereinabove) are utilized with $P_c/P_e = .1$ being assumed for detection of the minimum carbon concentration. The average of $\sqrt[6]{a_c^6}$ was estimated from the Coulter counter data to be $14.5\mu$. Substituting this into equations 8 and 9 yield $n_c = 141 \text{ cm}^{-3}$. This corresponds to a mass density of $n_c V_p = 2.17 \times 10^{-6}$ gm/ml, which is within a factor of two of the actual minimum concentration detected and corresponds to one carbon particle per 35 million erythrocytes.

There are several areas for possible error in this estimate. First, the Coulter counter data gives an approximate curve for the size distribution of the carbon particles. This causes errors in the computation of $a_c^6$. Second, the largest carbon particles have a diameter of $80\mu$. The wavelength of ultrasound m water at 10 MHz is $150\mu$. Therefore, the Rayleigh scattering assumption $(d<<\lambda)$ does not completely hold for these particles.

The backscattered power did not rise linearly with carbon concentration. This is probably due to mutual interference between the scatterers. At the lowest carbon concentration detected, the scatterers are more than one wavelength apart on the average, so that interference signal cancellation effects should be negligible. However, at the high concentrations shown in FIGURE 6, the average distance between scatterers is less than one wavelength, so interference effects can be expected to reduce the backscattered power to less than the sum of the powers scattered from each individual particle.

As can be appreciated from the foregoing, impurities such as carbon particles can be detected in real time in blood using the non-traumatic ultrasonic method and apparatus of this invention to monitor the backscatter power from an ultrasonic Doppler flowmeter.

This invention makes it possible to detect smaller concentrations of scatterers or smaller changes in the concentration of ultrasound scatterers for a given transmitted ultrasound power, than was previously possible. This is particularly advantageous when looking for possible contaminants in blood (such as microscopic particles of carbon which might be introduced into the blood coming from a kidney dialysis machine, for example, in which an accidental break in the dialysis membrane has occurred) since the invention makes it possible to detect the presence of contaminant particles in the blood using the minimum transmitted ultrasound power.

Minimizing the required power makes it possible to use this invention for battery operated undersea systems which are used for sea water flow measurement, since minimizing the required battery power of such systems makes it possible to leave such systems unattended for longer periods.

In addition, Doppler system sensitivity is maximized. Since the power transmitted by a Doppler system must be large enough so that the backscattered signal is larger than the thermal noise of the Doppler receiver, the Doppler system sensitivity is maximized when the Doppler output signal is compressed into the smallest possible bandwidth. For uniform velocity scatterers irradiated by a Doppler system with a small bandwidth, the Doppler output bandwidth depends on the range of angles $\Delta\phi$ over which sound rays emitted from the transducer can be backscattered to the transducer. The Doppler output bandwidth $B_d$ is minimized if $\Delta\phi$ is minimized. This occurs when the transducer is focused with focal length which is equal or larger than the near field distance of the transducer. Under these circumstances, the fractional bandwidth of the output Doppler signal $B_d/f_d$ approach $\lambda/D$ where $F_d, \lambda/D$ are respectively the center frequency of the output Doppler signal, the transmitted ultrasound wavelength and transducer diameter. Thus, the sensitivity of ultrasound Doppler systems is maximized by narrowing the output bandwidth by minimizing the range of angles $\Delta\phi$ over which rays leaving the transducer are backscattered to it, and this is specifically accomplished by transmitting ultrasound in short bursts and by focusing the transducer in its far field.

X-5628                          -22-

CLAIMS

1. A method for detecting impurities in a liquid wherein:

ultrasound Doppler radiation of narrow bandwidth is directed at flow of liquid and the radiation reflected from the scattering agents in the liquid is received;

the power levels of the received reflected radiation due to the scattering agents is found; and

from the power levels, impurities in the liquid are determined.

2. The method of Claim 1 wherein the ultrasound Doppler radiation is directed at an angle at the flow of liquid. .

3. The method of Claim 1 or 2 wherein ultrasound Doppler radiation is directed at a flow of liquid through a transducer.

4. The method of Claim 3 wherein the radiation is directed at a flow of liquid through a transducer focused in the far field.

5. The method of Claim 1, 2 or 3 wherein the flow of liquid is a flow of blood.

6. The method of Claim 5 wherein higher power levels relative to other scattering agents in a flow of blood are used to determine the presence of carbon particles in the blood.

7. The method of Claim 6 wherein carbon particles of about $2 \times 10^{-6}$ gm/ml of blood are determined from the higher power levels.

8.  A method of any one of Claims 1 to 7 wherein a spectrum averager is used to analyze and average the backscatter power in order to determine the presence of impurities.

9.  A method of any one of Claims 1 to 8 wherein the method includes filtering of the received radiation to filter out signals due to stationary echos.

10.  A device for detecting impurities in a liquid wherein the device comprises:

ultrasound Doppler means including transducer means for directing radiation of narrow bandwidth towards a flow of liquid and receiving reflected radiation therefrom, the Doppler means providing an output signal having a component indicative of impurities sensed in the flow of liquid; and

analyzing means for receiving the output signal from the Doppler means, for determining the presence of the impurities indicative of impurities included in the liquid and providing an indication of the presence of the impurities in the flow of liquid.

11.  The device of Claim 10 wherein the transducer means is focused in the far field.

12.  The device of Claim 10 wherein the transducer is positioned at an angle with respect to the flow of liquid.

13.  The device of Claim 10 wherein the analyzing means includes a power detector.

14.  The device of Claim 13 wherein the power detector is a spectrum analyzer.

15. The device of Claim 14 wherein the analyzing means includes an averaging means.

16. The device of Claim 15 wherein the averaging means includes a spectrum averager.

17. The device of any one of Claims 10 to 16 wherein the flow of liquid is a flow of blood, wherein the impurities to be detected include carbon particles, and wherein the analyzing means indicates the sensed presence of carbon particles in a flow of blood.

18. A device of any one of Claims 10 to 17 for detecting impurities in the blood wherein the device comprises:

ultrasonic Doppler flowmeter means including transducer means focused in the far field for directing radiation of narrow bandwidth towards a flow of blood and receiving radiation therefrom reflected from scattering agents in the blood, the flowmeter means providing an output signal having a component indicative of impurities acting as scattering agents in the flow of blood; and

analyzing means for receiving the output signal from the flowmeter means and determining therefrom average power levels due to the scattering agents including average power levels due to the impurities acting as scattering agents in the blood, an indication of the presence of impurities in the flow of blood.

FIG. 1

**FIG. 2**

FIG. 3

FIG. 4

FIG. 5

FIG. 6